# EUROPEAN PATENT APPLICATION

(11) **EP 4 413 941 A1**
(43) Date of publication of application: **14.08.2024**
(21) Application number: 22867448.7
(22) Date of filing: 09.09.2022
(51) Int. Cl.: A61B 34/30

(54) **INFERENCE DEVICE, INFORMATION PROCESSING METHOD, AND COMPUTER PROGRAM**

(30) Priority: 10.09.2021 US 202163242628 P
(71) Applicant: Anaut Inc., Tokyo 105-0022 (JP)
(72) Inventor: KOBAYASHI, Nao, Tokyo 105-0022 (JP); KUMAZU, Yuta, Tokyo 105-0022 (JP); SENYA, Seigo, Tokyo 105-0022 (JP)
(74) Representative: Cabinet Beaumont
(86) International application number: PCT/JP2022/033958
(87) International publication number: WO 2023/038127

(57) **Abstract**

An inference device, an information processing method, and a computer program are provided.

An inference device connected between a surgical robot and a console controlling the surgical robot, the inference device includes an image acquisition unit acquiring an operative field image shot by an imaging unit of the surgical robot, an inference unit performing an inference process on the acquired operative field image, and a transmission unit transmitting at least one of the operative field image acquired by the image acquisition unit and information based on an inference result by the inference unit to the console according to transmission settings by the console.

## Description

### [Technical Field]

The present invention relates to an inference device, an information processing method, and a computer program.

### [Background Art]

In recent years, surgery using a surgical robot has been performed on patients. In this type of surgical robot, two forceps used for surgery are attached to two robot arms, respectively. In addition, an affected part is imaged by an endoscope, and a three-dimensional image (an image that provides three-dimensional vision using parallax between the left eye and the right eye) of the affected part is displayed on a monitor. An operator, such as a doctor, operates an operation unit with both hands while referring to the monitor to manipulate the forceps attached to each arm.

### [Prior Art Document]

### [Patent Document]

[Patent Document 1] Japanese Patent Laid-Open Publication No. 2014-38075

### [Summary of Invention]

### [Problems to be Solved by Invention]

Patent Document 1 does not disclose a technique that presents an inference result for an operative field image to an operator.

An object of the present invention is to provide an inference device, an information processing method, and a computer program that can perform inference on an operative field image obtained from a surgical robot and transmit information based on an inference result to a console.

### [Means for Solving Problems]

According to an aspect of the present invention, there is provided an inference device connected between a surgical robot and a console controlling the surgical robot. The inference device includes: an image acquisition unit acquiring an operative field image shot by an imaging unit of the surgical robot; an inference unit performing an inference process on the acquired operative field image; and a transmission unit transmitting at least one of the operative field image acquired by the image acquisition unit and information based on an inference result by the inference unit to the console according to transmission settings by the console.

According to another aspect of the present invention, there is provided an information processing method executed by a computer connected between a surgical robot and a console controlling the surgical robot. The information processing method includes: acquiring an operative field image shot by an imaging unit of the surgical robot; performing inference on the acquired operative field image; and transmitting at least one of the operative field image and information based on an inference result to the console according to transmission settings received through the console.

According to still another aspect of the present invention, there is provided a computer program causing a computer connected between a surgical robot and a console controlling the surgical robot to execute a process including: acquiring an operative field image shot by an imaging unit of the surgical robot; performing inference on the acquired operative field image; and transmitting at least one of the operative field image and information based on an inference result to the console according to transmission settings received through the console.

### [Effects of Invention]

According to the present application, it is possible to perform inference on an operative field image obtained from a surgical robot and to transmit information based on an inference result to a console.

### [Brief Description of Drawings]

FIG. 1 is a block diagram illustrating an example of a configuration of a surgical robot system according to Embodiment 1.
FIG. 2 is a schematic diagram illustrating an example of an operative field image.
FIG. 3 is a schematic diagram illustrating an example of a configuration of a learning model.
FIG. 4 is a schematic diagram illustrating an example of an inference image.
FIG. 5 is a schematic diagram illustrating an example of display in a console.
FIG. 6 is a flowchart illustrating a procedure of a process performed in the surgical robot system according to Embodiment 1.
FIG. 7 is a flowchart illustrating a procedure of a process performed in a surgical robot system according to Embodiment 2.
FIG. 8 is a diagram illustrating a first specific example of a control method.
FIG. 9 is a diagram illustrating a second specific example of the control method.
FIG. 10 is a diagram illustrating a third specific example of the control method.
FIG. 11 is a diagram illustrating a fourth specific example of the control method.
FIG. 12 is a diagram illustrating a fifth specific example of the control method.
FIG. 13 is a diagram illustrating a sixth specific example of the control method.
FIG. 14 is a flowchart illustrating a procedure of a process performed by an inference unit in Embodiment 3.
FIG. 15 is a flowchart illustrating a procedure of a process performed by an inference unit in Embodiment 4.
FIG. 16 is a flowchart illustrating a procedure of a process performed by an inference unit in Embodiment 5.

### [Mode for Carrying out Invention]

Hereinafter, the present invention will be specifically described on the basis of the drawings illustrating embodiments of the present invention.

### (Embodiment 1)

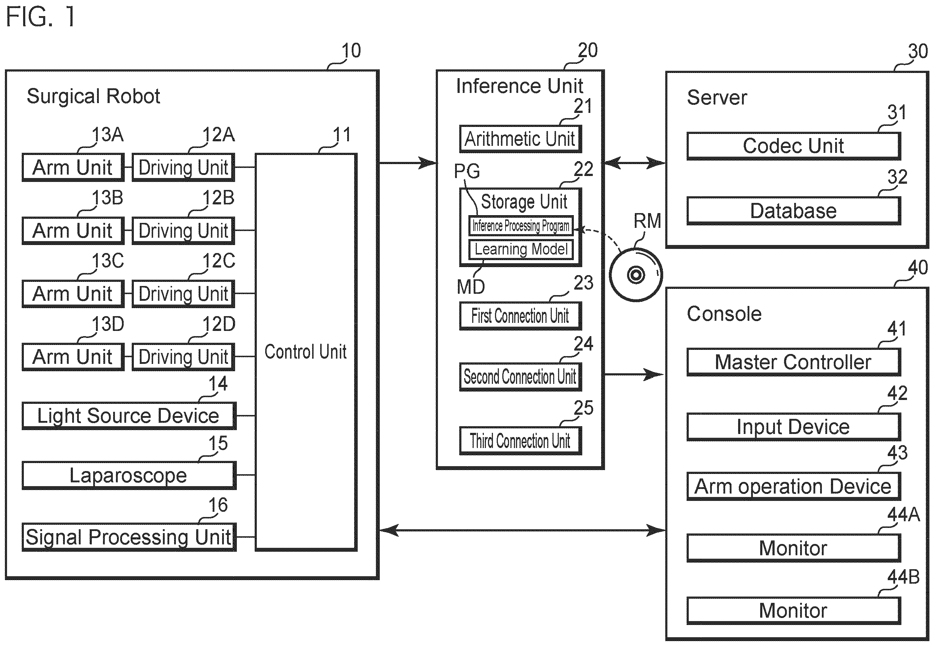

FIG. 1 is a block diagram illustrating an example of a configuration of a surgical robot system 1 according to Embodiment 1. The surgical robot system 1 according to Embodiment 1 includes a surgical robot 10, an inference unit 20, a server 30, and a console 40. In the surgical robot system 1, an operative field is imaged by a laparoscope 15 mounted on the surgical robot 10, and an operative field image obtained by the laparoscope 15 is displayed on monitors 44A and 44B of the console 40. An operator (doctor) performs laparoscopic surgery by moving an arm operation device 43 while checking the operative field image displayed on the monitors 44A and 44B to operate a surgical device mounted on the surgical robot 10.

In addition, the present invention is not limited to the laparoscopic surgery and can be applied to all robot-assisted endoscopic surgeries using thoracoscopes, gastrointestinal endoscopes, cystoscopes, arthroscopes, spinal endoscopes, neuroendoscopes, surgical microscopes, and the like.

Hereinafter, a configuration of each of the surgical robot 10, the inference unit 20, the server 30, and console 40 will be described.

The surgical robot 10 includes a control unit 11, driving units 12A to 12D, arm units 13A to 13D, a light source device 14, the laparoscope 15, a signal processing unit 16, and the like.

The control unit 11 is composed of, for example, a central processing unit (CPU), a read only memory (ROM), a random access memory (RAM), and the like. The control unit 11 controls the operation of each hardware unit included in the surgical robot 10 on the basis of, for example, control information input from the console 40.

One (arm unit 13A) of the arm unit 13A to 13D included in the surgical robot 10 is used to three-dimensionally move the laparoscope 15. Therefore, the laparoscope 15 is attached to a tip of the arm unit 13A. The driving unit 12A includes an actuator, a motor, and the like for driving the arm unit 13A and drives the arm unit 13A under the control of the control unit 11 to three-dimensionally move the laparoscope 15 attached to the tip. In addition, control for the movement of the laparoscope 15 may be automatic control or manual control through the console 40.

The remaining three arm units (arm units 13B to 13D) are used to three-dimensionally move the surgical devices. Therefore, the surgical devices are attached to the tips of the arm units 13B to 13D. The surgical devices include forceps, energy treatment tools, vascular clips, automatic anastomosis devices, and the like. The driving unit 12B includes an actuator, a motor, and the like for driving the arm unit 13B and drives the arm unit 13B under the control of the control unit 11 to three-dimensionally move the surgical device attached to the tip. The same applies to the driving units 12C and 12D. In addition, control for the movement of the surgical device is mainly manual control through the console 40. However, automatic control may also be used auxiliary. Further, the three arm units 13B to 13D do not need to be controlled at the same time, and two of the three arm units 13B to 13D are appropriately selected and manually controlled.

The light source device 14 includes a light source, a light guide, an illumination lens, and the like. The light source device 14 guides illumination light emitted from the light source to a tip of the light guide and irradiates the operative field with the illumination light through the illumination lens provided at the tip of the light guide. The light emitted by the light source device 14 may be normal light or special light. The normal light is, for example, light having a wavelength band of white light (380 nm to 650 nm). On the other hand, the special light is illumination light different from the normal light and corresponds to narrowband light, infrared light, excitation light, or the like.

The laparoscope 15 includes an imaging element, such as a complementary metal oxide semiconductor (CMOS), and a driver circuit provided with a timing generator (TG), an analog signal processing circuit (AFE), and the like. The driver circuit of the laparoscope 15 receives signals of each of R, G, and B output from the imaging element in synchronization with a clock signal output from the TG, and the AFE performs necessary processes, such as noise removal, amplification, and AD conversion, to generate digital image data (operative field image).

The signal processing unit 16 includes a digital signal processor (DSP), an image memory, and the like and performs appropriate processing, such as color separation, color interpolation, gain correction, white balance adjustment, and gamma correction, on the image data input from the laparoscope 15. The signal processing unit 16 generates frame images for a moving image from the processed image data and sequentially outputs each of the generated frame images to the inference unit 20. The frame rate of the frame image is, for example, 30 frames per second (FPS). For example, the signal processing unit 16 may output video data based on a predetermined standard such as National Television System Committee (NTSC), Phase Alternating Line (PAL), or Digital Imaging and COmmunication in Medicine (DICOM).

The inference unit 20 includes an arithmetic unit 21, a storage unit 22, a first connection unit 23, a second connection unit 24, a third connection unit 25, and the like.

The arithmetic unit 21 is composed of a CPU, a ROM, a RAM, and the like. The ROM in the arithmetic unit 21 stores, for example, a control program for controlling the operation of each hardware unit included in the inference unit 20. The CPU in the arithmetic unit 21 executes the control program stored in the ROM or a computer program stored in the storage unit 22, which will be described below, to control the operation of each hardware unit such that the entire device functions as an inference device according to the present application. The RAM in the arithmetic unit 21 temporarily stores, for example, data used during execution of computation.

In this embodiment, the arithmetic unit 21 is configured to include the CPU, the ROM, and the RAM. However, the arithmetic unit 21 may have any configuration and may be an arithmetic circuit or a control circuit including a graphics processing unit (GPU), a digital signal processor (DSP), a field programmable gate array (FPGA), a quantum processor, a volatile or nonvolatile memory, or the like. Further, the arithmetic unit 21 may have the functions of a clock that outputs date and time information, a timer that measures the time elapsed from the giving of a measurement start instruction to the giving of a measurement end instruction, a counter that counts numbers, and the like.

The storage unit 22 includes a storage device such as a flash memory. The storage unit 22 stores the computer program executed by the arithmetic unit 21, various types of data acquired from the outside, various types of data generated in the device, and the like.

The computer program stored in the storage unit 22 includes, for example, an inference processing program PG for causing the arithmetic unit 21 to perform an inference process on the operative field image. These computer programs may be a single computer program or a program group constructed by a plurality of computer programs. Further, the computer program including the inference processing program PG may be distributed and arranged in a plurality of computers and may be executed by the plurality of computers in cooperation with each other.

The computer program including the inference processing program PG is provided by a non-transitory recording medium RM on which the computer program is recorded to be readable. The recording medium RM is a portable memory such as a CD-ROM, a USB memory, or a secure digital (SD) card. The arithmetic unit 21 reads a desired computer program from the recording medium RM using a reading device (not illustrated) and stores the read computer program in the storage unit 22. Alternatively, the computer program including the inference processing program PG may be provided by communication. In this case, the arithmetic unit 21 downloads a desired computer program through communication and stores the downloaded computer program in the storage unit 22.

Furthermore, the storage unit 22 stores a learning model MD that is used for the inference process. An example of the learning model MD is a learning model used to infer the position of an object to be recognized in the operative field image. In this case, the learning model MD is configured to output information indicating the position of the object in a case where the operative field image is input. Here, the object to be recognized in the operative field image may be an organ, such as the esophagus, the stomach, the large intestine, the pancreas, the spleen, the ureter, the lung, the prostate, the uterus, the gallbladder, the liver, or the vas deferens, or may be a tissue, such as blood, a connective tissue, fat, a nerve, a blood vessel, a muscle, or a membranous structure. In addition, the object may be a surgical device, such as forceps, an energy treatment tool, a blood vessel clip, or an automatic anastomosis device. The learning model MD may output, as the information indicating the position of the target object, information of the probability indicating whether or not each pixel or each specific region corresponds to the object. The storage unit 22 stores definition information of the learning model MD including trained parameters.

Another example of the learning model MD is a learning model that is used to infer a scene. In this case, the learning model MD is configured to output information related to the scene shown by a surgical image in a case the operative field image is input. The information related to the scene output by the learning model MD is, for example, information of the probability of being a scene including a specific organ, the probability of being a scene in which a characteristic manipulation is performed during surgery, and the probability of being a scene in which a characteristic operation (ligation of vessels, cutting of the intestinal tract, anastomosis, or the like) using a specific surgical device (a vascular clip, an automatic anastomosis device, or the like).

Only one learning model MD is illustrated in FIG. 1 for simplicity. However, a plurality of learning models may be stored in the storage unit 22. For example, the storage unit 22 may store a plurality of learning models corresponding to a plurality of types of organs in order to recognize each organ or may store a plurality of learning models corresponding to organs and other structures in order to recognize the organs and the structures. Further, the storage unit 22 may store a learning model for recognizing a structure, such as an organ, and a learning model for recognizing a scene.

The first connection unit 23 includes a connection interface for connecting the surgical robot 10. The image data of the operative field image, which has been shot by the laparoscope 15 and processed by the signal processing unit 16, is input to the inference unit 20 through the first connection unit 23. The image data input through the first connection unit 23 is output to the arithmetic unit 21 and the storage unit 22.

The second connection unit 24 includes a connection interface for connecting the server 30. The inference unit 20 outputs the image data of the operative field image acquired by the surgical robot 10 and the inference result obtained by the arithmetic unit 21 to the server 30 through the second connection unit 24.

The third connection unit 25 includes a connection interface for connecting the console 40. The inference unit 20 outputs the image data of the operative field image acquired by the surgical robot 10 and the inference result obtained by the arithmetic unit 21 to the console 40 through the third connection unit 25. In addition, control information related to the surgical robot 10 may be input to the inference unit 20 through the third connection unit. The control information related to the surgical robot 10 includes, for example, information of the positions, angles, speeds, accelerations, and the like of the arm units 13A to 13D.

The inference unit 20 may include an operation unit that is composed of various switches and levers operated by the operator or the like. Predetermined specific functions or functions set by the operator may be assigned to the switches and the levers included in the operation unit. The inference unit 20 may include a display unit that displays information to be notified to the operator or the like in the form of text or images or may include an output unit that outputs the information to be notified to the operator or the like by voice or sound.

The server 30 includes a codec unit 31, a database 32, and the like. The codec unit 31 has a function of encoding the image data of the operative field input from the inference unit 20 and storing the encoded image data in the database 32, a function of reading the image data stored in the database 32 and decoding the image data, and the like. The database 32 stores the image data encoded by the codec unit 31.

The console 40 includes a master controller 41, an input device 42, the arm operation device 43, the monitors 44A and 44B, and the like.

The master controller 41 is composed of a CPU, a ROM, a RAM, and the like and controls the operation of each hardware unit included in the console 40. The input device 42 is an input device, such as a keyboard, a touch panel, a switch, or a lever, and receives instructions and information input by the operator or the like. The input device 42 is mainly a device for operating the inference unit 20 and may be configured to select an object to be operated in order to receive the switching of a display function of the console 40.

The arm operation device 43 includes an operation tool for remotely operating the arm units 13A to 13B of the surgical robot 10. The operation tool includes a left-hand operation lever that is operated by the left hand of the operator and a right-hand operation lever that is operated by the right hand of the operator. The arm operation device 43 measures the movement of the operation tool using a measurement device, such as a rotary encoder, and outputs a measured value to the master controller 41. The master controller 41 generates control instructions for controlling the arm units 13A to 13D of the surgical robot 10 on the basis of the measured value input from the arm operation device 43 and transmits the generated control instructions to the surgical robot 10. The surgical robot 10 controls the operation of the arm units 13A to 13D on the basis of the control instructions input from the console 40. Therefore, the arm units 13A to 13D of the surgical robot 10 are configured to operate following the movement of the operation tools (the left-hand operation lever and the right-hand operation lever) in the console 40.

The monitors 44A and 44B are display devices such as liquid crystal displays for displaying necessary information to the operator. For example, one of the monitors 44A and 44B is used as a main monitor for displaying the operative field image, and the other is used as a sub-monitor for displaying supplementary information such as patient information. Further, when the laparoscope 15 is configured to output an operative field image for the left eye and an operative field image for the right eye, the operative field image may be three-dimensionally displayed by displaying the operative field image for the left eye on the monitor 44A and displaying the operative field image for the right eye on the monitor 44B.

In the example of the configuration illustrated in FIG. 1, the inference unit 20 and the server 30 are provided separately. However, the inference unit 20 and the server 30 may be configured as an integrated device. Furthermore, the inference unit 20 and the server 30 may be integrated into the console 40.

Next, the operative field image input to the inference unit 20 will be described.

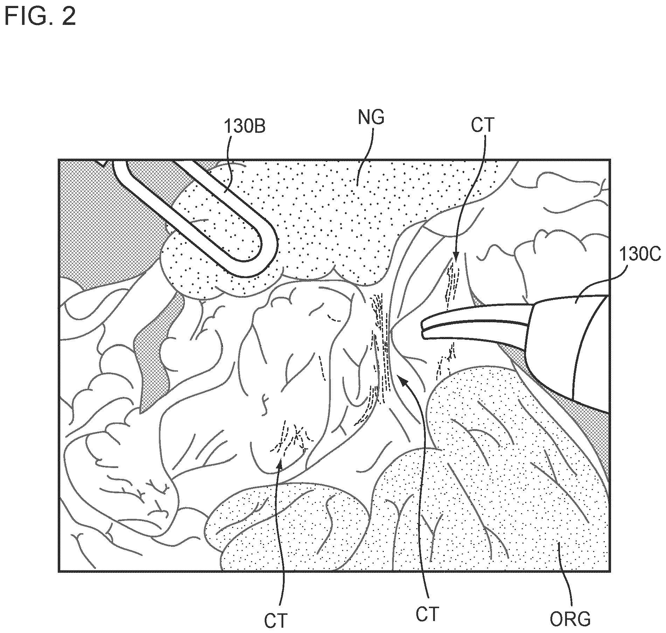

FIG. 2 is a schematic diagram illustrating an example of the operative field image. The operative field image in this embodiment is an image obtained by imaging the inside of the abdominal cavity of a patient with the laparoscope 15. The operative field image does not need to be a raw image output by the laparoscope 15, but may be an image (frame image) processed by the signal processing unit 16 or the like.

The operative field imaged by the laparoscope 15 includes various tissues such as organs, blood vessels, nerves, connective tissues, lesions, membranes, and layers. The operator cuts a tissue including a lesion using a surgical tool, such as an energy treatment tool or forceps, while ascertaining the relationship between these anatomical structures. The operative field illustrated in FIG. 2 includes a tissue NG that includes a lesion, such as a malignant tumor, a tissue ORG that constitutes an organ, and a connective tissue CT that connects these tissues. In this embodiment, the tissue NG is a part to be removed from the body, and the tissue ORG is a part to be left in the body. In the example illustrated in FIG. 2, the connective tissue CT is exposed by grasping the tissue NG with forceps 130B and expanding the tissue NG to the upper side of FIG. 2.

In laparoscopic surgery, for example, surgery is performed to remove a lesion such as a malignant tumor formed in the body of the patient. At this time, the operator grasps the tissue NG including the lesion with the forceps 130B and expands the tissue NG in an appropriate direction such that the connective tissue CT present between the tissue NG including the lesion and the tissue ORG to be left is exposed. The operator excises the exposed connective tissue CT using an energy treatment tool 130C to separate the tissue NG including the lesion from the tissue ORG to be left.

The inference unit 20 acquires the operative field image illustrated in FIG. 2 and performs the inference process on the acquired operative field image. Specifically, the inference unit 20 infers the position of the object to be recognized in the operative field image. The learning model MD is used for the inference process.

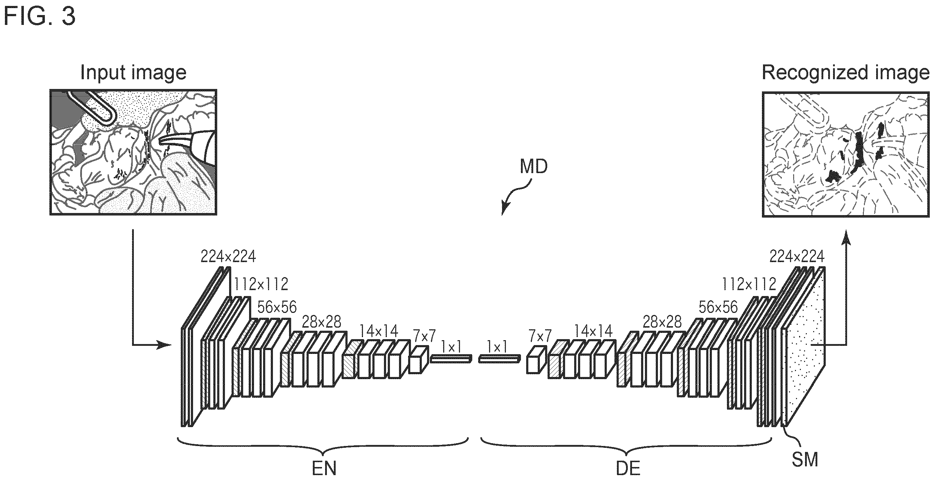

FIG. 3 is a schematic diagram illustrating an example of a configuration of the learning model MD. The learning model MD is a learning model for performing image segmentation and is constructed by, for example, a neural network including a convolutional layer such as SegNet. The learning model MD is not limited to SegNet and may be constructed using any neural network that can perform the image segmentation, such as Fully Convolutional Network (FCN), U-Shaped Network (U-Net), and Pyramid Scene Parsing Network (PSPNet). In addition, the learning model MD may be constructed using a neural network for object detection, such as You Only Look Once (YOLO) or Single Shot Multi-Box Detector (SSD), instead of the neural network for image segmentation.

The learning model MD includes, for example, an encoder EN, a decoder DE, and a softmax layer SM. The encoder EN is configured by alternately arranging convolutional layers and pooling layers. The convolutional layer is divided into multiple layers, for example, 2 to 3 layers. In the example illustrated in FIG. 3, the convolutional layer is not hatched, and the pooling layer is hatched.

The convolutional layer performs a convolution operation between input data and a filter with a predetermined size (for example, 3 × 3 or 5 × 5). That is, the convolutional layer multiplies an input value input to the position corresponding to each element of the filter by a weighting coefficient set in advance in the filter for each element to compute a linear sum of the multiplied values for each element. A set bias is added to the computed linear sum to obtain an output of the convolutional layer. In addition, the result of the convolution operation may be converted by an activation function. For example, a rectified linear unit (ReLU) can be used as the activation function. The output of the convolutional layer indicates a feature map obtained by extracting the features of the input data.

The pooling layer computes local statistics of the feature map output from the convolutional layer which is an upper layer connected to the input side. Specifically, a window with a predetermined size (for example, 2 × 2 or 3 × 3) corresponding to the position of the upper layer is set, and local statistics are computed from the input value in the window. For example, the maximum value can be used as the statistics. The size of the feature map output from the pooling layer is reduced (down-sampled) according to the size of the window. In the example illustrated in FIG. 3, the encoder EN sequentially repeats the operation of the convolutional layer and the operation of the pooling layer to sequentially down-sample an input image of 224 pixels × 224 pixels into 112 × 112, 56 × 56, 28 × 28, ..., 1 × 1 feature maps.

The output (the 1 × 1 feature map in the example illustrated in FIG. 3) of the encoder EN is input to the decoder DE. The decoder DE is configured by alternately arranging deconvolutional layers and depooling layers. The deconvolutional layer is divided into multiple layers, for example, 2 to 3 layers. In the example illustrated in FIG. 3, the deconvolutional layer is not hatched, and the depooling layer is hatched.

The deconvolutional layer performs a deconvolution operation on an input feature map. The deconvolution operation is an operation that restores the feature map before the convolution operation, on the presumption that the input feature map is the result of the convolution operation using a specific filter. In this operation, when the specific filter is represented by a matrix, the product of a transposed matrix for the matrix and the input feature map is computed to generate an output feature map. In addition, the operation result of the deconvolutional layer may be converted by the above-described activation function such as ReLU.

The depooling layers included in the decoder DE are individually associated with the pooling layers included in the encoder EN on a one-to-one basis, and the associated pairs have substantially the same size. The depooling layer re-increases (up-samples) the size of the feature map down-sampled in the pooling layer of the encoder EN. In the example illustrated in FIG. 3, the decoder DE sequentially repeats the operation of the convolutional layer and the operation of the pooling layer to sequentially up-sample the feature map into 1 × 1, 7 × 7, 14 × 14, ..., 224 × 224 feature maps.

The output (the 224 × 224 feature map in the example illustrated in FIG. 3) of the decoder DE is input to the softmax layer SM. The softmax layer SM applies a softmax function to the input value from the deconvolutional layer connected to the input side to output the probability of a label that identifies a part at each position (pixel). The learning model MD according to this embodiment may output, from the softmax layer SM, the probability that each pixel will correspond to the object to be recognized in a case where the operative field image is input.

The arithmetic unit 21 of the inference unit 20 can extract a pixel for which the probability of the label output from the softmax layer SM is equal to or greater than a threshold value (for example, equal to or greater than 90%) with reference to the computation result by the learning model MD to generate an image (inference image) indicating the position of the object to be recognized.

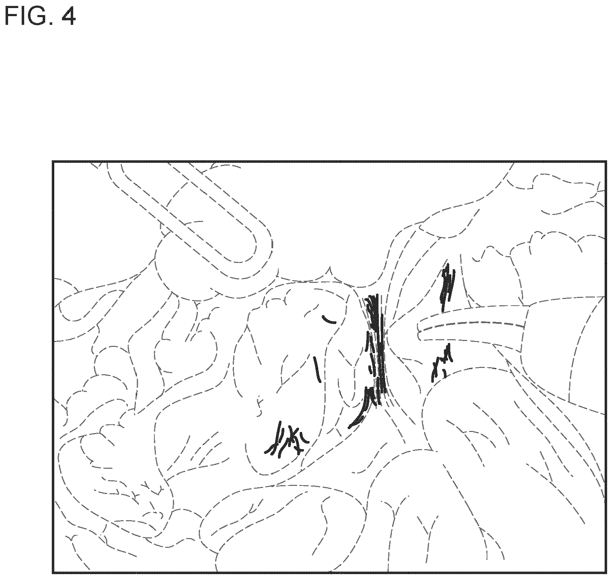

FIG. 4 is a schematic diagram illustrating an example of the inference image. The example illustrated in FIG. 4 is an inference image indicating the position of the connective tissue. In FIG. 4, a connective tissue portion inferred using the learning model MD is represented by a thick solid line, and the other organ and tissue portions are represented by broken lines for reference. The arithmetic unit 21 of the inference unit 20 generates an inference image of the connective tissue in order to display the inferred connective tissue portion to be distinguishable. The inference image is an image that has the same size as the operative field image and is an image in which a specific color is assigned to a pixel inferred as the connective tissue. It is preferable that the color assigned to the pixel of the connective tissue is a color that is not present inside the human body such that it can be distinguished from the organs, the blood vessels, and the like. The color that is not present inside the human body is, for example, a cool (bluish) color such as blue or aqua. Further, a transparency level is set for each pixel constituting the inference image. The pixel recognized as the connective tissue is set to be opaque, and the other pixels are set to be transparent. The inference image generated in this way is displayed to be superimposed on the operative field image such that the connective tissue portion can be displayed as a structure having a specific color on the operative field image.

In the example illustrated in FIG. 4, the inference image of the connective tissue is displayed. However, the object to be recognized is not limited to the connective tissue and may be any structure such as an organ, blood (bleeding), or a surgical device. In this embodiment, it is assumed that the object to be recognized is set in advance and the learning model MD for the object is trained in advance and stored in the storage unit 22.

The inference unit 20 transmits at least one of the operative field image (also referred to as an original image) acquired from the surgical robot 10 and the inference image generated from the operative field image to the console 40. The image to be transmitted is set through the input device 42 of the console 40. That is, when transmission is set such that both the original image and the inference image are transmitted, the inference unit 20 transmits both the original image and the inference image to the console 40. When transmission is set such that only the original image (or only the inference image) is transmitted, the inference unit 20 transmits only the original image (or only the inferred image) to the console 40.

In this embodiment, the inference image showing the position of the object in the operative field image is generated, and the generated inference image is transmitted to the console 40. However, instead of the configuration in which the inference image is transmitted, a configuration may be adopted in which positional information indicating the position of the object in the operative field image is generated and the generated positional information is transmitted to the console 40. Here, the positional information indicating the position of the object may be information for designating a pixel corresponding to the object or may be information for designating, for example, the contour or center of gravity of a region. In addition, in a case where the inference image is transmitted, one-way communication from the inference unit 20 to the console 40 may be used. In a case where the positional information is used, two-way communication between the inference unit 20 and the console 40 may be used. Further, the original image and the inference image (or the positional information) may be transmitted to the server 30 and stored in the database 32.

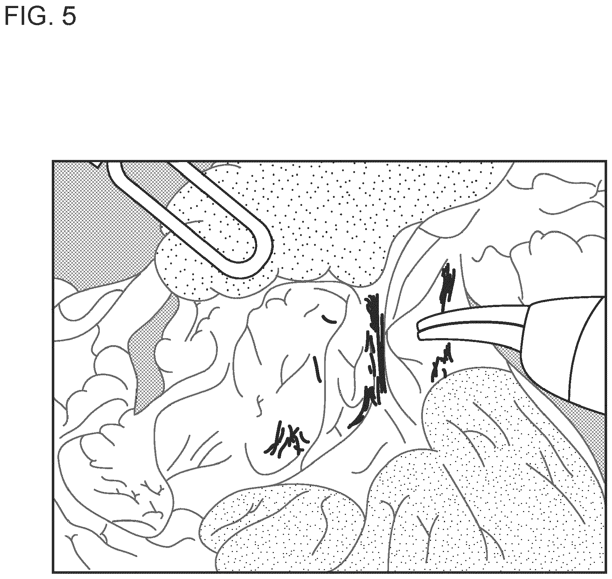

The console 40 receives the operative field image and the inference image transmitted from the inference unit 20 and displays the images on the monitors 44A and 44B. FIG. 5 is a schematic diagram illustrating an example of display in the console 40. The console 40 can display the inference image on the monitor 44A (or monitor 44B) to be superimposed on the operative field image. The example of the display illustrated in FIG. 5 is an example in which the inference image of the connective tissue is displayed to be superimposed on the original image. For convenience of illustration, the connective tissue portion is represented by a thick solid line. However, in practice, each pixel of a portion corresponding to the connective tissue is painted in a color that is not present in the human body, such as blue or aqua. Therefore, the operator can check a display screen to clearly identify the connective tissue and to understand the part to be excised.

In this embodiment, the inference image is displayed on the monitor 44A (or the monitor 44B) to be superimposed on the operative field image. However, the operative field image may be displayed in one region of the display screen, and the inference image may be displayed in the other region. In addition, the operative field image may be displayed on one monitor 44A, and the inference image may be displayed on the other monitor 44B.

In a case where the console 40 receives the positional information indicating the position of the object in the operative field image from the inference unit 20, the console 40 may generate the inference image of the object on the basis of the positional information and display the generated inference image on the monitors 44A and 44B to be superimposed on the operative field image (or independently of the operative field image).

Hereinafter, the operation of the surgical robot system 1 will be described.

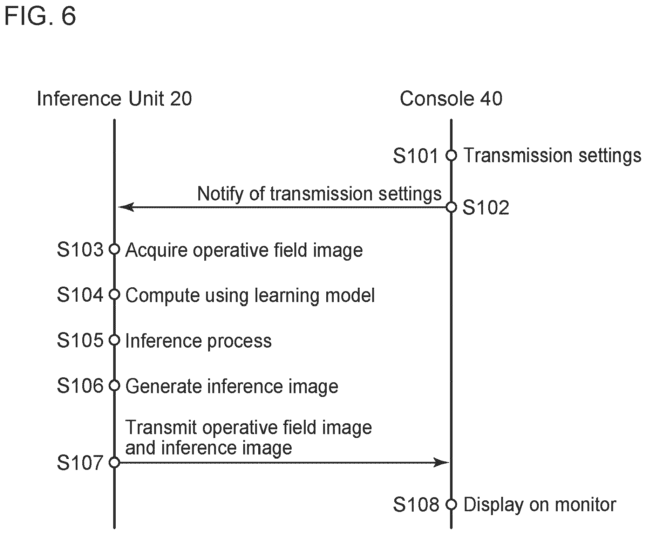

FIG. 6 is a flowchart illustrating a procedure of a process performed in the surgical robot system 1 according to Embodiment 1. The console 40 receives transmission settings for the operative field image and the inference image through the input device 42 (step S101). In the transmission settings, settings for whether to transmit only the operative field image, only the inferred image, or both the operative field image and the inferred image are received. The console 40 notifies the inference unit 20 of the received transmission settings (step S102).

When the imaging of the operative field by the laparoscope 15 in the surgical robot 10 is started, the inference unit 20 acquires an operative field image through the first connection unit 23 (step S103). The arithmetic unit 21 of the inference unit 20 performs computation on the acquired operative field image using the learning model MD (step S104) and performs the inference process on the operative field image (step S105). The arithmetic unit 21 acquires the inference result from the learning model MD and generates an inference image as information based on the inference result (step S106). Instead of the configuration in which the inference image is generated, positional information indicating the position of the object may be generated. The arithmetic unit 21 may perform the processes in steps S104 to S106 each time an operative field image is acquired in units of frames in step S103. However, in a case where transmission is set such that only the operative field image is transmitted, the arithmetic unit 21 may omit the processes in steps S104 to S106.

The inference unit 20 transmits at least one of the operative field image and the inference image to the console 40 according to the transmission settings received in step S102 (step S107). Further, the inference unit 20 may perform a process of transmitting at least one of the operative field image and the inference image to the server 30 and storing the image in the database 32.

In a case where the console 40 receives at least one of the operative field image and the inference image transmitted from the inference unit 20, the console 40 displays the received image on the monitors 44A and 44B (step S108). In a case where the console 40 receives both the operative field image and the inference image, the console 40 displays the operative field image and the inference image on the monitor 44A (or the monitor 44B) such that the inference image is superimposed on the operative field image. Alternatively, the console 40 may separately display the operative field image and the inference image on the monitors 44A and 44B. In a case where the console 40 receives either the operative field image or the inference image, the console 40 displays the received image on the monitor 44A (or the monitor 44B).

As described above, in Embodiment 1, the transmission settings from the inference unit 20 to the console 40 can be made according to the determination of the operator who operates the console 40, and at least one of the operative field image and the inference image can be transmitted from the inference unit 20 to the console 40 according to the transmission settings. Therefore, in a scene in which the inference image is not required, it is possible to stop the transmission of the inference image and to reduce a communication load between the inference unit 20 and the console 40.

### (Embodiment 2)

In Embodiment 2, a configuration will be described in which the inference unit 20 generates control information of the surgical robot 10 and the surgical robot is controlled through the console 40.

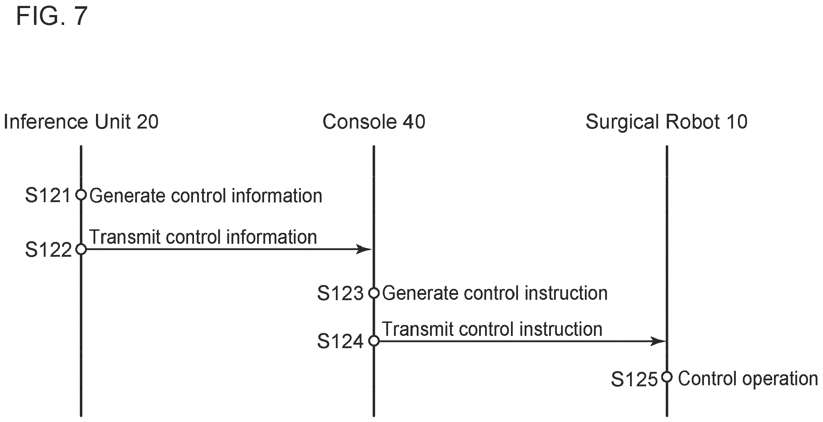

FIG. 7 is a flowchart illustrating a procedure of a process performed in a surgical robot system 1 according to Embodiment 2. The surgical robot system 1 performs, for example, an inference process on the operative field image, a process of transmitting an image from the inference unit 20 to the console 40, and a process of displaying the image on the monitors 44A and 44B through the same procedure as that in Embodiment 1.

After performing the inference process, the arithmetic unit 21 of the inference unit 20 generates control information for controlling the operation of the surgical robot 10 according to the inference result (step S121) and transmits the generated control information to the console 40 (step S122).

For example, the arithmetic unit 21 may recognize a tip portion of the energy treatment tool 130C chronologically on the basis of the inference result of the learning model MD and compute the amount of control (the amount of movement, a rotation angle, a speed, the amount of change in angular velocity, and the like) of the arm unit 13A holding the laparoscope 15 such that the laparoscope 15 is moved following the tip portion of the energy treatment tool 130C. In addition, the arithmetic unit 21 may recognize a type of surgical device set in advance by the operator using the learning model MD, generate control information so as to automatically follow the recognized surgical device, and transmit the control information to the console 40.

Further, the arithmetic unit 21 may compute the area of the object chronologically on the basis of the inference result of the learning model MD and compute the amount of control of the arm unit 13A holding the laparoscope 15 such that the laparoscope 15 is moved following the portion in which the computed area increases or decreases. Here, the object may be the lesion or connective tissue to be excised or may be blood (bleeding) or the like.

Furthermore, the arithmetic unit 21 may recognize the object or the shape of the object on the basis of the inference result of the learning model MD and compute the amount of control of the arm unit 13A holding the laparoscope 15 such that the laparoscope 15 is moved to a designated position on the recognized object. The object is, for example, a specific organ. In addition, the designated position on the object may be the center of gravity of the object or may be any point on the periphery of the object.

Moreover, the arithmetic unit 21 may compute a distance between the laparoscope 15 and the object (mainly the distance in a depth direction) on the basis of the inference result of the learning model MD and compute the amount of control of the arm unit 13A holding the laparoscope 15 according to the computed distance. Specifically, the arithmetic unit 21 may compute the distance of the arm unit 13A such that the computed distance is a preset distance.

In addition, the control unit 21 may compute the amount of control of the arm unit 13A holding the laparoscope 15 so as to follow a region in which the confidence of the inference result is relatively high.

Further, the amount of control of the arm unit 13A is computed as the amount of change from the current position, angle, speed, and angular velocity of the arm unit 13A. The arithmetic unit 21 can acquire information of the current position, angle, speed, and angular velocity of the arm unit 13A from the console 40. The arithmetic unit 21 may recognize the object from the inference result of the learning model MD and compute the amount of change in the position, angle, speed, angular velocity, and the like of the arm unit 13A according to the position of the recognized object or displacement from the previous recognized position.

In a case where the master controller 41 of the console 40 receives control information of the surgical robot 10 from the inference unit 20, the master controller 41 generates control instructions for the surgical robot 10 on the basis of the received control information (step S123). The control instructions are configured by, for example, one or more instructions that are predetermined between the surgical robot 10 and the console 40. The console 40 transmits the control instructions generated by the master controller 41 to the surgical robot 10 (step S124).

In a case where the control unit 11 of the surgical robot 10 receives the control instructions transmitted from the console 40, the control unit 11 drives the driving units 12A to 12D in response to the received control instructions to control the operation of the arm units 13A to 13D (step S125).

As described above, in Embodiment 2, it is possible to control the operation of the surgical robot 10 according to the inference result of the inference unit 20.

Hereinafter, a specific example of a method for controlling the surgical robot 10 in Embodiment 2 will be disclosed.

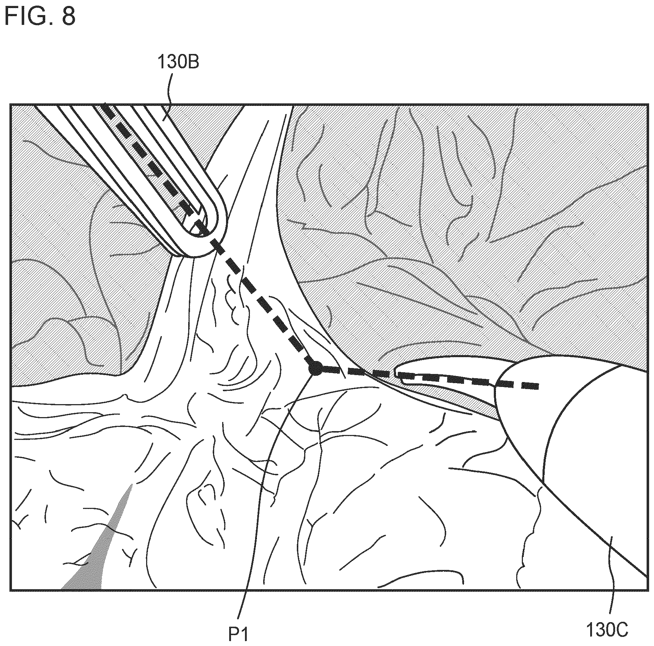

(1) FIG. 8 is a diagram illustrating a first specific example of the control method. In many cases, a region that the operator wants to see in the operative field image is the vicinity of an intersection point between an extension line of the surgical device operated by the dominant hand (for example, the right hand) and an extension line of the surgical device operated by the non-dominant hand (for example, the left hand). Therefore, the arithmetic unit 21 recognizes the surgical device operated by the dominant hand and the surgical device operated by the non-dominant hand on the basis of the inference result of the learning model MD, derives the extension line of each surgical device, and computes the intersection point between the extension lines. In the example illustrated in FIG. 8, the extension line of each surgical device is represented by a broken line, and the intersection point is represented by P1. The arithmetic unit 21 computes the amount of control of the arm unit 13A holding the laparoscope 15 such that an imaging center of the laparoscope 15 is matched with the intersection point P1 and transmits control information based on the computation result to the console 40. The console 40 can automatically control the operation of the arm unit 13A on the basis of the control information from the inference unit 20.

Instead of automatically controlling the arm unit 13A, the console 40 may start the control in a case where a trigger operation is received from the operator. A gesture motion with the surgical device can be adopted as the trigger operation. For example, in a case where the console 40 receives a predetermined gesture motion, such as a motion of moving the tip of the surgical device close to the intersection point P1 or a motion of pointing the surgical device to the intersection point P1, the console 40 may determine that the trigger operation has been received and start the control of the operation of the arm unit 13A. The control may be started in a case where a predetermined input operation by the input device 42 (a touch operation on a touch panel, the input of a command by a keyboard, or the like) is received, instead of the gesture motion with the surgical device. Further, in a case where the inference unit 20 or the console 40 includes a voice input unit, the control of the operation of the arm unit 13A may be started using the input of a predetermined voice as a trigger.

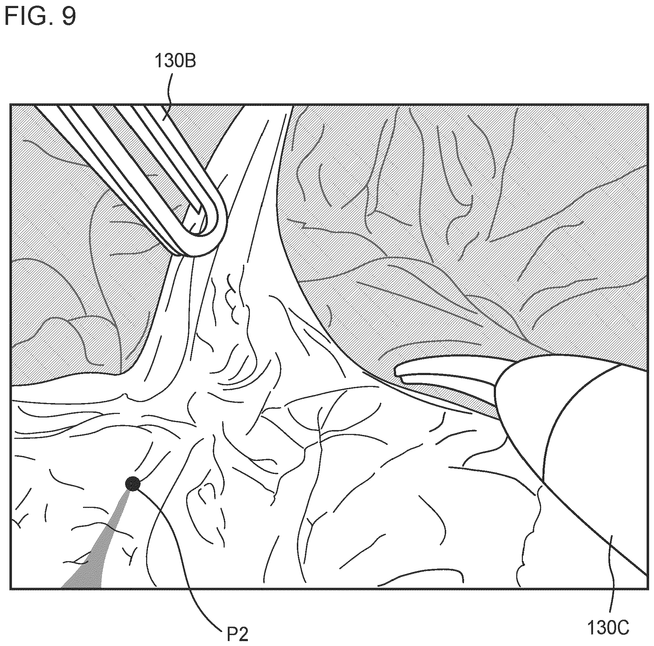

(2) FIG. 9 is a diagram illustrating a second specific example of the control method. In the operative field image, one of the parts that the operator wants to check is a tip portion of the blood vessel. Therefore, the arithmetic unit 21 recognizes the shape of the blood vessel appearing in the operative field image on the basis of the inference result of the learning model MD and specifies a portion with a tapered shape to specify the tip portion of the blood vessel. In the example illustrated in FIG. 9, the tip portion of the blood vessel is represented by P2. The arithmetic unit 21 compute the amount of control of the arm unit 13A holding the laparoscope 15 such that the imaging center of the laparoscope 15 is matched with the tip portion P2 of the blood vessel and transmits control information based on the computation result to the console 40.

In addition, the imaging center of the laparoscope 15 is not always matched with the tip portion of the blood vessel. Therefore, in a case where the trigger operation by the operator is received, the console 40 may start the control of the operation of the arm unit 13A. The trigger operation is the same as that in the first specific example. That is, a predetermined gesture motion with the surgical device, a predetermined input operation with the input device 42, the input of a predetermined voice, or the like can be used as the trigger operation.

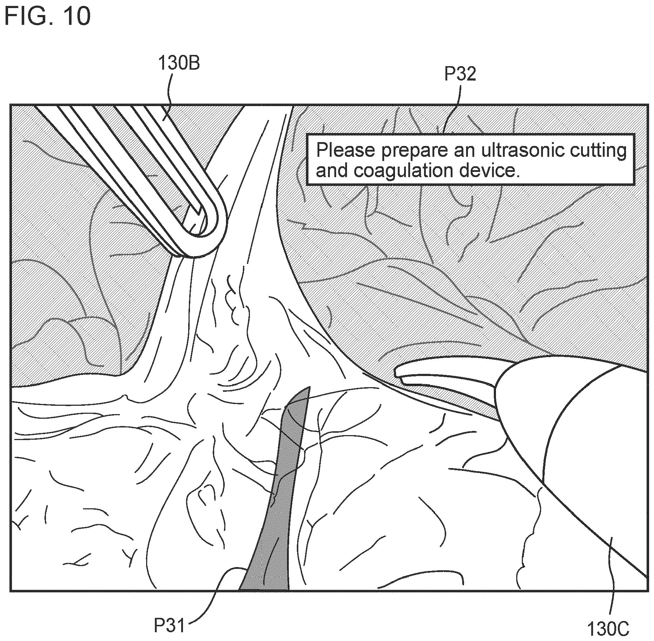

(3) FIG. 10 is a diagram illustrating a third specific example of the control method. In a case where the object to be treated by the surgical device is a blood vessel, an appropriate surgical device is selected according to the area (thickness) of the blood vessel, the shape of the blood vessel, and the like. Therefore, the arithmetic unit 21 recognizes the blood vessel appearing in the operative field image on the basis of the inference result of the learning model MD and computes the area of the blood vessel from the recognition result. At this time, the arithmetic unit 21 may normalize the area of the blood vessel, using the size (area) of the surgical device (the forceps 130B or the energy treatment tool 130C) included in the operative field image as a reference. In addition, the arithmetic unit 21 may recognize the shape of the blood vessel appearing in the operator image on the basis of the inference result of the learning model MD. The arithmetic unit 21 selects an appropriate surgical device (for example, a 5-mm clip, a 10-mm clip, an ultrasonic cutting and coagulation device, or the like) according to the computed area of the blood vessel or the recognized shape of the blood vessel and notifies the console 40 of information of the selected surgical device. The console 40 displays the information of the surgical device notified from the inference unit 20 on, for example, the monitor 44A. FIG. 10 illustrates an example in which a shape P31 of a blood vessel appearing in the operative field image is recognized and text information P32 of "Please, prepare an ultrasonic cutting and coagulation device" is displayed on the monitor 44A. Instead of the configuration in which the text information is displayed, an icon may be displayed, or the operator may be notified by voice. In addition, in a case where there is an arm unit (for example, the arm unit 13D) that is not operated by the dominant hand or non-dominant hand of the operator and a target surgical device (ultrasonic cutting and coagulation device or the like) is attached to this arm unit, the operator may be prompted to switch the arm unit.

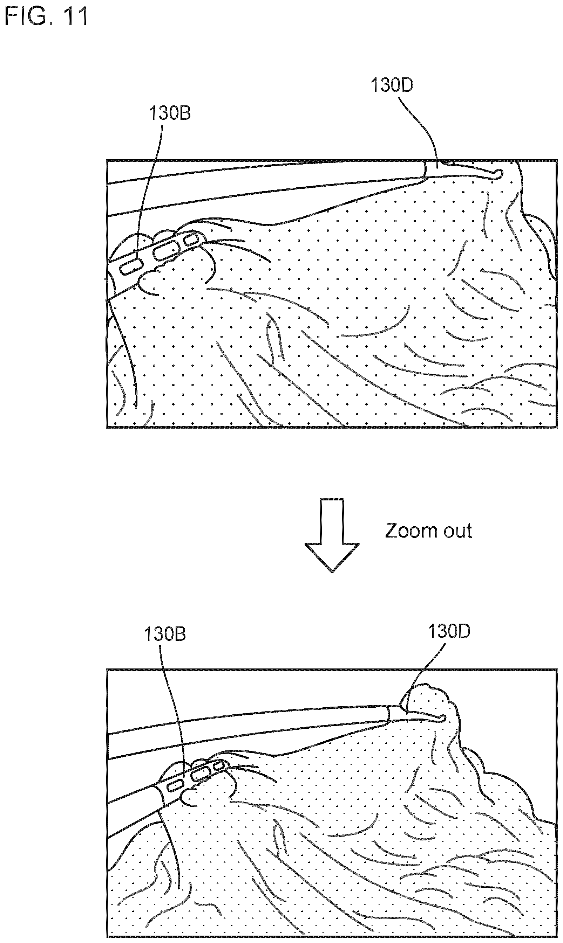

(4) FIG. 11 is a diagram illustrating a fourth specific example of the control method. In a scene in which both the surgical devices operated by the dominant hand and non-dominant hand of the operator are grasping forceps and some tissues are expanded, it is preferable to zoom out to a range in which the grasping forceps are sufficiently visible. Therefore, the arithmetic unit 21 recognizes the surgical devices operated by the dominant hand and non-dominant hand of the operator on the basis of the inference result of the learning model MD and determines whether both the surgical devices are grasping forceps. In a case where the arithmetic unit 21 determines that both the surgical devices are grasping forceps, the arithmetic unit 21 computes the amount of control of the arm unit 13A holding the laparoscope 15 so as to zoom out to a range in which the grasping forceps are sufficiently visible and transmits control information based on the computation result to the console 40. Alternatively, the arithmetic unit 21 may compute depth information and compute the amount of control of the arm unit 13A holding the laparoscope 15 on the basis of the computed depth information. In the example illustrated in FIG. 11, a scene in which an adipose tissue is grasped using two grasping forceps 130B and 130D is illustrated, and a state in which zooming-out is performed with the recognition of the two grasping forceps 130B and 130D is illustrated.

In the fourth specific example, in a case where the learning model MD recognizes that both the surgical devices are grasping forceps, zooming-out is performed. However, in a case where the operator attempts to move the third arm (for example, the arm unit 13D) to which the grasping forceps are attached, zooming-out may be performed. Further, in the fourth specific example, zooming-out is performed by moving the arm unit 13A holding the laparoscope 15. However, in a case where the laparoscope 15 has a zoom function, the zoom function of the laparoscope 15 may be controlled to zoom out.

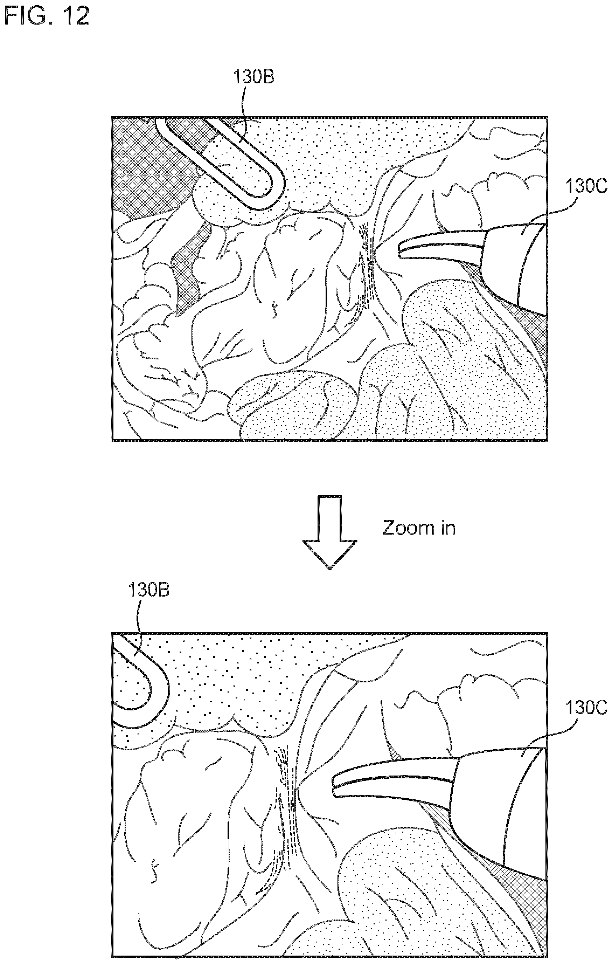

(5) FIG. 12 is a diagram illustrating a fifth specific example of the control method. In a scene in which the operator operates a cutting device to cut a target tissue, it is preferable to zoom in to a range in which the tip of the cutting device is sufficiently visible. Therefore, the arithmetic unit 21 recognizes the surgical device operated by the dominant hand of the operator on the basis of the inference result of the learning model MD and determines whether the recognized surgical device is the cutting device. In a case where the arithmetic unit 21 determines that the surgical device operated by the dominant hand of the operator is the cutting device, the arithmetic unit 21 computes the amount of control of the arm unit 13A holding the laparoscope 15 so as to zoom in to a range in which the tip of the cutting device is sufficiently visible and transmits control information based on the computation result to the console 40. For example, the arithmetic unit 21 may compute the area of a tip portion of the cutting device and compute the amount of control of the arm unit 13A in order to zoom in such that the computed area is equal to or greater than a set value. In the example illustrated in FIG. 12, a state in which, as a result of recognizing that the surgical device operated by the dominant hand of the operator is the energy treatment tool 130C (cutting device), the tip portion of the energy treatment tool 130C is zoomed in is illustrated.

In addition, after zooming in, the console 40 may automatically control the operation of the arm unit 13A so as to follow the tip of the cutting device. Alternatively, the console 40 may control the operation of the arm unit 13A so as to follow the tip of the cutting device and perform zooming-in in a case where the tip of the cutting device is stationary. In the fifth specific example, zooming-in is performed by moving the arm unit 13A holding the laparoscope 15. However, in a case where the laparoscope 15 has a zoom function, a zoom mechanism of the laparoscope 15 may be controlled to zoom in.

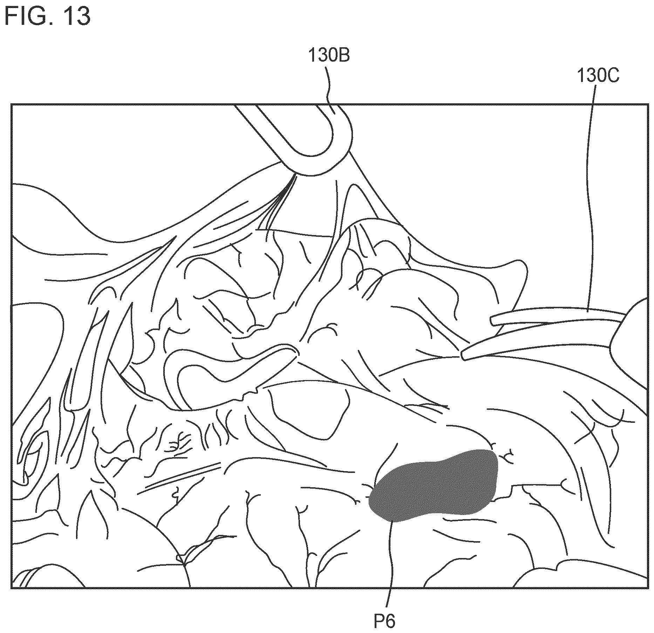

(6) FIG. 13 is a diagram illustrating a sixth specific example of the control method. In a case where bleeding is detected, control to move the laparoscope 15 or control to place gauze may be performed. The arithmetic unit 21 recognizes a bleeding region on the basis of the inference result of the learning model MD and computes the area of the recognized bleeding region. In the example illustrated in FIG. 13, the bleeding region is represented by P6. The area of bleeding region P6 corresponds to the amount of bleeding. The arithmetic unit 21 determines whether the computed area of the bleeding region P6 is equal to or greater than a preset threshold value. In a case where the arithmetic unit 21 determines that the area is equal to or greater than the threshold value, the arithmetic unit 21 computes the amount of control of the arm unit 13A holding the laparoscope 15 such that the imaging center of the laparoscope 15 is a point in the bleeding region P6 (for example, the center of gravity of the bleeding region P6) and transmits control information based on the computation result to the console 40.

The arithmetic unit 21 may transmit, to the console 40, control information for performing control to place gauze in the bleeding region P6, instead of the control to move the laparoscope 15 (or together with the control to move the laparoscope 15). Specifically, in a case where grasping forceps for grasping gauze are attached to the arm unit 13D, the arithmetic unit 21 may generate control information for controlling the operation of the arm unit 13D and transmit the control information to the console 40. In addition, the console 40 may display text information indicating that gauze should be placed in the bleeding region P6 on the monitor 44A.

The arithmetic unit 21 may perform control to move the laparoscope 15 in a case where the amount of bleeding is relatively small and may perform control to place gauze in a case where the amount of bleeding is relatively large. For example, a first threshold value and a second threshold value (however, the first threshold value < the second threshold value) may be set for the area of the bleeding region. In a case where the area of the bleeding region P6 is equal to or greater than the first threshold value, the control to move the laparoscope 15 may be performed. In a case where the area of the bleeding region P6 is equal to or greater than the second threshold value, the control to place gauze may be performed.

Further, the console 40 may start the above-described control in a case where a trigger operation by the operator is received. The trigger operation is the same as that in the first specific example. That is, a predetermined gesture motion with the surgical device, a predetermined input operation with the input device 42, the input of a predetermined voice, or the like can be used as the trigger operation.

### (Embodiment 3)

In Embodiment 3, a configuration will be described in which the resolution of the operative field image is changed in accordance with the confidence of the inference result.

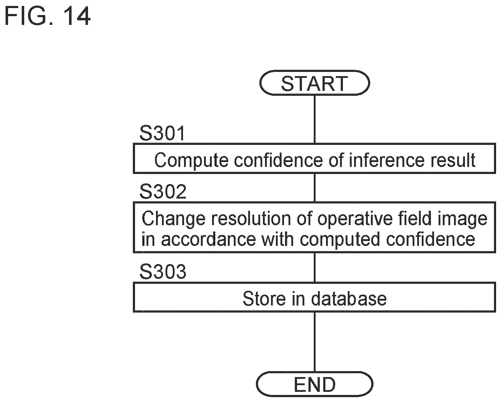

FIG. 14 is a flowchart illustrating a procedure of a process performed by the inference unit 20 in Embodiment 3. The surgical robot system 1 performs the inference process on the operative field image through the same procedure as that in Embodiment 1.

After performing the inference process, the arithmetic unit 21 of the inference unit 20 computes the confidence of the inference result (step S301). The confidence of the inference result is computed on the basis of the probability output from the softmax layer SM of the learning model MD. For example, the arithmetic unit 21 can compute the average of the probability values for each pixel estimated to be the object to compute the confidence.

The arithmetic unit 21 changes the resolution of the operative field image in accordance with the computed confidence (step S302). The arithmetic unit 21 may set the resolution Y (dpi: dot per inch) of the operative field image as Y = (X - 100)/k (k is a constant) for the confidence X (X = 0 to 100%) and change the resolution of the operative field image in accordance with the set resolution Y. Alternatively, the arithmetic unit 21 may change the resolution to a preset resolution in a case where the confidence is less than a threshold value.

The arithmetic unit 21 transmits the operative field image or the inference image whose resolution has been changed to the server 30 and stores the image in the database 32 (step S303).

As described above, in Embodiment 3, it is possible to change the resolution of the operative field image which has low confidence and in which it is difficult to determine whether or not the object is present and to save storage capacity.

### (Embodiment 4)

In Embodiment 4, a configuration will be described in which a score of surgery performed by the surgical robot is computed on the basis of the confidence of the inference result and information of the surgical robot 10.

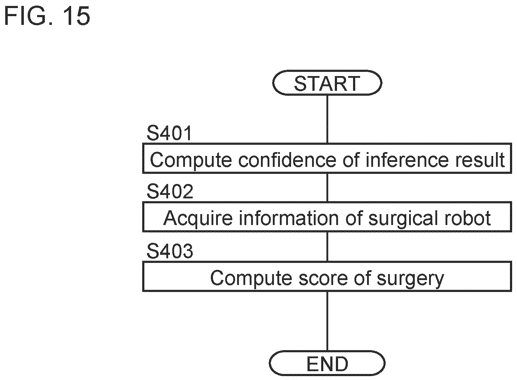

FIG. 15 is a flowchart illustrating a procedure of a process performed by the inference unit 20 in Embodiment 4. The surgical robot system 1 performs the inference process on the operative field image through the same procedure as that in Embodiment 1.

After performing the inference process, the arithmetic unit 21 of the inference unit 20 computes the confidence of the inference result (step S401). The confidence of the inference result is computed on the basis of the probability output from the softmax layer SM of the learning model MD. For example, the arithmetic unit 21 can compute the average of the probability values for each pixel estimated to be the object to compute the confidence.

The arithmetic unit 21 acquires the information of the surgical robot 10 from the console 40 (step S402). For example, the arithmetic unit 21 may acquire information of the position, angle, speed, angular velocity, and the like of the arm units 13A to 13D.

The arithmetic unit 21 computes the score of the surgery on the basis of the confidence computed in step S401 and the information of the surgical robot 10 acquired in step S402 (step S403). A function or a learning model that is configured to output the score of the surgery in response to the input of the confidence and the information of the surgical robot 10 is prepared in advance. The confidence and the information of the surgical robot 10 can be input to the function or the learning model to compute the score. In addition, the arithmetic unit 21 may compute the score on the basis of information, such as the confidence of an anatomical structure (object), the area thereof, an increase or decrease in the area, operation information of the surgical device, and a recognition result (trajectory or the like) of the surgical device, using a function or a learning model prepared in advance. Further, the arithmetic unit 21 may determine the next operation of the surgical robot 10 or present the next operation to the operator on the basis of the computed score.

### (Embodiment 5)

A surgical robot system 1 according to Embodiment 5 is a system that generates operative field images for the left eye and the right eye with the laparoscope 15 and outputs the generated operative field images for the left eye and the right eye to the monitors 44A and 44B through the inference unit 20 to perform three-dimensional display.

In the surgical robot system 1 according to Embodiment 5, the arithmetic unit 21 of the inference unit 20 performs the inference process on each of the operative field image for the left eye and the operative field image for the right eye. An inference procedure is the same as that in Embodiment 1.

In addition, the arithmetic unit 21 can compute the confidence of the inference result for each of the operative field image for the left eye and the operative field image for the right eye. A method for computing the confidence is the same as that in Embodiment 3. In a case where the confidences of the inference results for the left eye and the right eye are different from each other, the arithmetic unit 21 can output an alert.

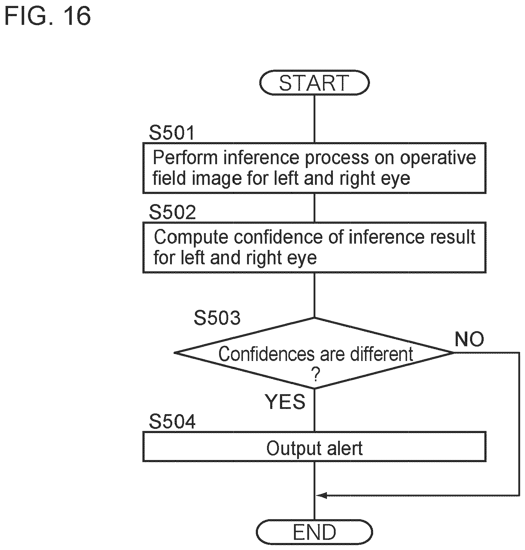

FIG. 16 is a flowchart illustrating a procedure of a process performed by the inference unit 20 in Embodiment 5. The arithmetic unit 21 of the inference unit 20 performs the inference process on each of the operative field image for the left eye and the operative field image for the right eye (step S501). The inference procedure is the same as that in the Embodiment 1.

The arithmetic unit 21 computes the confidence of each inference result (step S502). A method for computing the confidence is the same as that in Embodiment 3.

The arithmetic unit 21 compares the confidence obtained from the operative field image for the left eye with the confidence obtained from the operative field image for the right eye to determine whether or not the confidences are different from each other (step S503). In a case where the difference between the confidences is equal to or greater than a predetermined percentage (for example, 10%), the arithmetic unit 21 determines that the confidences are different from each other.

In a case where it is determined that the confidences are different from each other (step S503: YES), the arithmetic unit 21 outputs an alert since the laparoscope 15 is likely to be inclined with respect to the object (step S504). Specifically, the arithmetic unit 21 transmits text information indicating that the laparoscope 15 is inclined to the console 40 to be displayed on the monitors 44A and 44B.

In Embodiment 5, an alert is output in a case where the confidences of the left and right sides are different from each other. However, the arithmetic unit 21 may generate control information for making the laparoscope 15 directly face the object and transmit the generated control information to the console 40.

Further, the arithmetic unit 21 may compute depth information on the basis of the parallax between the operative field images for the left and right eyes and transmit the computed depth information to the console 40. Here, the arithmetic unit 21 may compute the depth information of a designated position. For example, the depth information of a designated position (the center of gravity, four corners, any point on the contour, a set position group, or the like) on the object may be computed.

Furthermore, the arithmetic unit 21 may generate control information for controlling the operation of the laparoscope 15 on the basis of the computed depth information and transmit the generated control information to the console 40. For example, in a case where the depth to the object is equal to or greater than a set value, the arithmetic unit 21 may generate control information for automatically zooming the laparoscope 15 and transmit the generated control information to the console 40. In addition, the arithmetic unit 21 may determine the point or route where the surgical device arrives on the basis of the depth information and automatically move the arm units 13A to 13D to the vicinity of the object to be excised. Then, in a case where the surgical device approaches the object to be excised, the arithmetic unit 21 may perform control to display information "Please excise" on the monitors 44A and 44B. Further, an alert may be output in a case where an attempt is made to excise a portion that should not be excised or in a case where a dangerous sign, such as bleeding, is detected.

The embodiments disclosed herein are illustrative in all respects and should be considered not to be restrictive. The scope of the present invention is not indicated by the above-described meaning, but is indicated by the claims. The present invention is intended to include all changes within the meaning and scope equivalent to the claims.

The matters described in each embodiment can be combined with each other. In addition, the independent claims and dependent claims recited in the claims may be combined with each other in all possible combinations, regardless of the form in which they are cited. Further, the claims are described in a format in which a claim refers to two or more other claims (multi-claim format). However, the present invention is not limited thereto. The claims will be described in a format (multi-multi claim) in which a multiple dependent claim refers to at least one multiple dependent claim.

### [Description of Reference Numerals]

- 10: Surgical robot
- 11: Control unit
- 12A to 12D: Driving unit
- 13A to 13D: Arm unit
- 14: Light source device
- 15: Laparoscope
- 16: Signal processing unit
- 20: Inference unit
- 21: Arithmetic unit
- 22: Storage unit
- 23: First connection unit
- 24: Second connection unit
- 25: Third connection unit
- 30: Server
- 31: Codec unit
- 32: Database
- 40: Console
- 41: Master controller
- 42: Input device
- 43: Arm operation device
- 44A, 44B: Monitor

## Claims

1. An inference device connected between a surgical robot and a console controlling the surgical robot, the inference device comprising:
an image acquisition unit acquiring an operative field image shot by an imaging unit of the surgical robot;
an inference unit performing an inference process on the acquired operative field image; and
a transmission unit transmitting at least one of the operative field image acquired by the image acquisition unit and information based on an inference result by the inference unit to the console according to transmission settings by the console.

2. The inference device according to claim 1,
wherein the inference unit infers at least one of a position of an object to be recognized in the operative field image and an event occurring in the operative field image.

3. The inference device according to claim 2,
wherein the inference unit generates image data indicating the position of the object, and
the transmission unit transmits the image data using one-way communication with the console.

4. The inference device according to claim 3,
wherein the inference unit generates positional information indicating the position of the object, and
the transmission unit transmits the positional information using two-way communication with the console.

5. The inference device according to any one of claims 2 to 4, further comprising:
a control unit generating control information for controlling an operation of the surgical robot according to the inference result by the inference unit,
wherein the transmission unit transmits the control information generated by the control unit to the console.

6. The inference device according to claim 5,
wherein the object is a surgical device, and
the control unit generates the control information in order to move the imaging unit following a tip portion of the recognized surgical device.

7. The inference device according to claim 5,
wherein the control unit computes an area of the recognized object and generates the control information in order to move the imaging unit following a portion in which the computed area increases or decreases.

8. The inference device according to claim 5,
wherein the control unit generates the control information in order to move the imaging unit to a designated position on the object.

9. The inference device according to claim 5,
wherein the control unit generates the control information in order to move the imaging unit according to a distance between the imaging unit and the object.

10. The inference device according to claim 5,
wherein the inference unit infers a motion or gesture of an operator operating the console, and
the control unit generates control information in order to control an operation of the imaging unit or a surgical device according to an inference result by the inference unit.

11. The inference device according to claim 5,
wherein the control unit computes an area or shape of the recognized object and generates control information for selecting or controlling a surgical device to be used according to the area or shape of the object.

12. The inference device according to any one of claims 1 to 4, further comprising:
a computation unit computing a confidence of the inference result by the inference unit; and
a resolution change unit changing a resolution of the operative field image according to the confidence computed by the computation unit.

13. The inference device according to any one of claims 1 to 4, further comprising:
a computation unit computing a confidence of the inference result by the inference unit; and
an information acquisition unit acquiring information of the surgical robot from the console,
wherein the computation unit computes a score of a surgery performed by the surgical robot on the basis of the computed confidence and the information acquired by the information acquisition unit.

14. The inference device according to any one of claims 1 to 4,
wherein the imaging unit of the surgical robot is configured to output an operative field image for a left eye and an operative field image for a right eye,
the image acquisition unit acquires the operative field image for the left eye and the operative field image for the right eye output from the imaging unit, and
the inference unit individually infers each of the acquired operative field image for the left eye and the acquired operative field image for the right eye.

15. The inference device according to claim 14, further comprising:
a computation unit computing a confidence of the inference result by the inference unit; and
an output unit outputting an alert on the basis of a difference between the confidence of the inference result computed for the operative field image for the left eye and the confidence of the inference result computed for the operative field image for the right eye.

16. The inference device according to claim 14, further comprising:
a computation unit computing a confidence of the inference result by the inference unit; and
a control unit generating control information for moving the imaging unit according to a difference between the confidence of the inference result computed for the operative field image for the left eye and the confidence of the inference result computed for the operative field image for the right eye,
wherein the transmission unit transmits the control information generated by the control unit to the console.

17. The inference device according to claim 14, further comprising:
a computation unit computing depth information on the basis of the operative field image for the left eye and the operative field image for the right eye,
wherein the transmission unit transmits the computed depth information to the console.

18. The inference device according to claim 14, further comprising:
a computation unit computing depth information on the basis of the operative field image for the left eye and the operative field image for the right eye; and
a control unit generating control information for controlling an operation of the surgical robot according to the computed depth information,
wherein the transmission unit transmits the control information generated by the control unit to the console.

19. An information processing method executed by a computer connected between a surgical robot and a console controlling the surgical robot, the information processing method comprising:
acquiring an operative field image shot by an imaging unit of the surgical robot;
performing inference on the acquired operative field image; and
transmitting at least one of the operative field image and information based on an inference result to the console according to transmission settings received through the console.

20. A computer program causing a computer connected between a surgical robot and a console controlling the surgical robot to execute a process comprising:
acquiring an operative field image shot by an imaging unit of the surgical robot;
performing inference on the acquired operative field image; and
transmitting at least one of the operative field image and information based on an inference result to the console according to transmission settings received through the console.
